# EUROPÄISCHE PATENTANMELDUNG

(11) **EP 3 485 916 A1**
(43) Veröffentlichungstag der Anmeldung: **22.05.2019**
(21) Anmeldenummer: 17202317.8
(22) Anmeldetag: 17.11.2017
(51) Int. Cl.: A61L 2/07, A61L 2/28, A61B 90/70, G01N 31/22, C12Q 1/22

(54) **VORRICHTUNG UND VERFAHREN ZUM NACHWEIS EINER ERFOLGREICHEN THERMISCHEN DESINFEKTION ODER STERILISATION**

(71) Anmelder: MELAG Medizintechnik oHG, 10829 Berlin (DE)
(72) Erfinder: PFEIFER, Stefan, 10823 Berlin (DE); BONATZ, Stefan, 12623 Berlin (DE); BECKER, Tino, 10117 Berlin (DE); PODOLSKI, Denis, 13585 Berlin (DE)
(74) Vertreter: Maikowski & Ninnemann Patentanwälte Partnerschaft mbB

(57) **Zusammenfassung**

Die Erfindung betrifft eine Vorrichtung zum Nachweis einer erfolgreichen thermischen Desinfektion oder Sterilisation, mit einer Prozessmedienleitung (11) und einer mit der Prozessmedienleitung (11) strömungstechnisch verbundenen Indikatoraufnahme (14) zur Aufnahme eines Indikators (17), der zum Anzeigen des Erfolgs einer thermischen Desinfektion oder Sterilisation dient. Die Vorrichtung zeichnet sich erfindungsgemäß dadurch aus, dass sie ein Verbindungsmittel zum direkten Verbinden mit einem zu desinfizierenden oder zu sterilisierenden Hohlkörperobjekt (2, 20) unter Ausbildung einer Strömungsverbindung zwischen der Prozessmedienleitung (11) und einem Innenlumen (21) des Hohlkörperobjekts (2, 20) aufweist, so dass ein der Vorrichtung (1, 10) zugeführtes Prozessmedium dann, wenn ein Hohlkörperobjekt (2, 20) mit der Vorrichtung (1, 10) verbunden ist, sowohl durch die Prozessmedienleitung (11) der Vorrichtung (1, 10) als auch durch das Innenlumen (21) des Hohlkörperobjekts (2, 20) geleitet wird. Die Erfindung betrifft ferner eine Anordnung aus einer solchen Vorrichtung und einem Hohlkörperobjekt, ein Gerät zur thermischen Desinfektion oder Sterilisation von Hohlkörperobjekten mit einer solchen Vorrichtung sowie ein Verfahren zur thermischen Desinfektion oder Sterilisation von Hohlkörperobjekten unter Einsatz einer derartigen Vorrichtung.

## Beschreibung

Die vorliegende Erfindung betrifft eine Vorrichtung zum Nachweis einer erfolgreichen thermischen Desinfektion oder Sterilisation gemäß dem Oberbegriff des Anspruchs 1, eine Anordnung aus einer solchen Vorrichtung und einem mit dieser Vorrichtung verbundenen zu desinfizierenden oder zu sterilisierenden Hohlkörperobjekt gemäß dem Oberbegriff des Anspruchs 7, ein Gerät zur thermischen Desinfektion oder Sterilisation von Hohlkörperobjekten gemäß dem Oberbegriff des Anspruchs 11 sowie ein Verfahren zur thermischen Desinfektion oder Sterilisation von Hohlkörperobjekten gemäß dem Oberbegriff des Anspruchs 13.

Um den Erfolg einer thermischen Desinfektion oder Sterilisation nachzuweisen, werden gemäß dem Stand der Technik speziell geformte Prüfkörper, sogenannte Helices oder Process Challenge Devices (PCDs), eingesetzt. Diese Prüfkörper simulieren regelmäßig die angenommenen schwierigsten Anforderungen an die Desinfektion oder Sterilisation von Hohlkörperinstrumenten, indem sie eine besondere Bauform oder Strömungsführung aufweisen. Um den Erfolg eines Sterilisationsvorgangs anzuzeigen, verwenden derartige Prüfkörper regelmäßig Chemoindikatoren, beispielsweise Chemoindikatoren der Klasse 5 nach DIN EN 11140. Wenn ein solcher Chemoindikator mit Dampf beaufschlagt wird, kommt es infolge einer chemischen Reaktion zu einem Farbumschlag, anhand dessen der Erfolg des Desinfektions- oder Sterilisationsvorgangs abgelesen werden kann.

Statt eines Chemoindikators kann auch ein Bioindikator eingesetzt werden. Ein solcher Bioindikator umfasst Mikroorganismen, deren Wachstumskinetik durch einen Sterilisations- oder Desinfektionsvorgang verändert wird. Anhand der veränderten Wachstumskinetik lässt sich dann die Qualität des durchgeführten Desinfektions- oder Sterilisationsvorgangs ermitteln.

Es ist auch möglich, das Erreichen der grundsätzlichen Sterilisationsbedingungen messtechnisch zu erfassen, beispielsweise mittels eines Temperatursensors. Dann ist es jedoch nicht möglich, die Innengeometrien der zu desinfizierenden oder zu sterilisierenden Hohlkörperobjekte zu berücksichtigen.

Aus dem Stand der Technik sind bereits verschiedene Möglichkeiten bekannt, wie die tatsächlichen Strömungsverhältnisse innerhalb der zu sterilisierenden oder zu desinfizierenden Hohlkörperobjekte nachgebildet werden können. So muss der üblicherweise als Sterilisationsmedium eingesetzte Wasserdampf bei einigen Prüfkörpern komplexe Strömungsführungen durchlaufen oder ein poröses Material oder eine Membran durchdringen, um zum Indikator zu gelangen; die DIN EN 13060 zeigt eine entsprechende Prüfkörperabbildung. Aber komplexe Strömungsführungen, poröse Materialien oder das Vorsehen von Membranen können stets nur die tatsächlichen Strömungsverhältnisse in den zu sterilisierenden Hohlkörperobjekten simulieren. Daher kann es für den Wasserdampf beispielsweise schwieriger sein, durch die vorgenannten Barrieren zu dem in einem Prüfkörper angeordneten Indikator zu gelangen, als die Hohlräume der zu sterilisierenden Hohlkörperobjekte zu durchdringen. Dann dauert es länger als eigentlich nötig, bis der Indikator eine erfolgreiche Sterilisation anzeigt. Daher müssen einige Sterilisationsverfahren länger als eigentlich notwendig durchgeführt werden, was neben einem Zeitverlust auch einen erhöhten Energieeintrag in ein solches Sterilisationssystem bedeutet.

Aus der DE 102 13 066 A1 ist eine Prüfvorrichtung für einen Sterilisationsvorgang bekannt, bei der ein mäanderförmiger Diffusionshohlraum vorgesehen ist, der von einem Sterilisationsmedium durchströmt werden muss, damit dieses Sterilisationsmedium zu einem am Ende des Diffusionshohlraums befindlichen Indikator gelangt.

Aus der US 2007/0264683 A1 ist ein Prüfkörper bekannt, bei dem ein Indikator von mehreren dampfdurchlässigen Lagen umgeben ist, die von Wasserdampf durchdrungen werden müssen, damit dieser in Kontakt mit dem Indikator treten kann.

Aus der EP 1 084 714 A1 ist eine Prüfvorrichtung bekannt, bei der ein Indikator über einen Strömungspfad mit einem Sterilisationsraum verbunden ist. Der Querschnitt des Strömungspfades kann über eine veränderliche Spielanpassung variiert werden, sodass die Schwierigkeit für das Sterilisationsmedium, den Indikator zu erreichen, einstellbar ist.

Aus der WO 01/56618 A1 ist eine Prüfvorrichtung bekannt, die aus zwei miteinander verbundenen Teilen besteht. In einem dieser Teile befindet sich eine Aufnahme für einen Chemoindikator. Der andere Teil besteht aus einem porösen Material das von dem Sterilisationsmedium durchströmt werden muss, um zu dem Chemoindikator zu gelangen.

Bei all diesen Prüfvorrichtungen handelt es sich um separate Bauteile, die einen bestimmten Bauraum innerhalb von Desinfektions- oder Sterilisationsgeräten benötigen. Darüber hinaus sind aus dem Stand der Technik auch Prüfvorrichtungen bekannt, die anstelle eines zu desinfizierenden oder zu sterilisierenden Hohlkörperobjekts mit einem zum Anbringen eines Hohlkörperobjekts vorgesehenen Adapter verbunden werden und somit die insgesamt zur Verfügung stehende Desinfektions- oder Sterilisationskapazität reduzieren.

Der vorliegenden Erfindung liegt die Aufgabe zugrunde, eine Vorrichtung zum Nachweis des Erfolgs einer thermischen Desinfektion oder Sterilisation bereitzustellen, die genauer als die aus dem Stand der Technik bekannten Vorrichtungen funktioniert und deren Einsatz die insgesamt zur Verfügung stehende Desinfektions- oder Sterilisationskapazität eines Geräts zur thermischen Desinfektion oder Sterilisation nicht reduziert.

Diese Aufgabe wird mit einer Vorrichtung zum Nachweis einer erfolgreichen thermischen Desinfektion oder Sterilisation mit den Merkmalen des Anspruchs 1 gelöst. Eine solche Vorrichtung weist eine Prozessmedienleitung auf, durch die sämtliche Prozessmedien während eines Aufbereitungs-, Desinfektions- oder Sterilisationsprozesses strömen können. Mit der Prozessmedienleitung ist eine Indikatoraufnahme strömungstechnisch verbunden. Die Indikatoraufnahme dient der Aufnahme eines Indikators, der in der Lage ist, den Erfolg einer thermischen Desinfektion oder Sterilisation anzuzeigen. Wie oben ausgeführt, kann es sich bei den in die Indikatoraufnahme einzusetzenden Indikatoren beispielsweise um Chemoindikatoren oder um Bioindikatoren handeln. Dabei ist eine Kompatibilität der Indikatoraufnahme mit Chemoindikatoren der Klasse 5 nach DIN EN 11140 besonders bevorzugt.

Die Vorrichtung zum Nachweis einer erfolgreichen thermischen Desinfektion oder Sterilisation zeichnet sich erfindungsgemäß dadurch aus, dass sie ein Verbindungsmittel aufweist, das zum direkten (unmittelbaren) Verbinden mit einem zu desinfizierenden oder zu sterilisierenden Hohlkörperobjekt dient. Dabei ist das Verbindungsmittel derart ausgestaltet, dass zwischen der Prozessmedienleitung und einem Innenlumen des Hohlkörperobjekts eine Strömungsverbindung hergestellt wird, wenn das Hohlkörperobjekt über das Verbindungsmittel mit der Vorrichtung verbunden wird. Dies hat zur Folge, dass ein Prozessmedium, das der Vorrichtung zugeführt wird immer dann, wenn ein Hohlkörperobjekt mit der Vorrichtung verbunden ist, sowohl durch die Prozessmedienleitung der Vorrichtung als auch durch das Innenlumen des Hohlkörperobjektes geleitet wird.

Mit dieser Variante lässt sich also eine feste Verbindung zwischen einem zu sterilisierenden oder zu desinfizierenden Hohlkörperobjekt und der Vorrichtung zum Nachweis einer erfolgreichen Sterilisation oder Desinfektion herstellen, sodass die Vorrichtung und das Objekt eine Einheit bilden. Auf diese Weise lässt sich besonders einfach sicherstellen, dass die Strömungsverhältnisse, die für ein Desinfektionsmedium oder Sterilisationsmedium, welches durch die Prozessmedienleitung strömt, maßgeblich von den innerhalb des Innenlumens des Hohlkörperobjekts vorherrschenden Strömungsbedingungen beeinflusst werden. Dann lässt sich über ein Auslesen eines in der Indikatoraufnahme angeordneten Indikators ein besonders genaues Ergebnis in Bezug auf die konkreten Desinfektions- bzw. Sterilisationsbedingungen innerhalb des Hohlkörperobjektes ermitteln. Durch die erfindungsgemäß beanspruchte Vorrichtung zum Nachweis einer erfolgreichen Desinfektion oder Sterilisation werden also nicht mehr die Strömungsverhältnisse innerhalb der zu desinfizierenden oder zu sterilisierenden Hohlkörperobjekte simuliert. Vielmehr werden die Anforderungen an das Desinfektions- oder Sterilisationsmedium zum Durchströmen der Vorrichtung durch die realen Strömungsverhältnisse innerhalb der zu desinfizierenden oder zu sterilisierenden Hohlkörperobjekte vorgegeben. Folglich hängt das anhand eines in der Indikatoraufnahme vorhandenen Indikators gewonnene Ergebnis hinsichtlich des Erfolgs der durchgeführten Desinfektion oder Sterilisation nicht mehr von bestimmten Annahmen, sondern lediglich von den konkreten Strömungsverhältnissen innerhalb der zu desinfizierenden oder zu sterilisierenden Hohlkörperobjekt ab.

Grundsätzlich ist es dabei unerheblich, ob die Vorrichtung zum Nachweis einer erfolgreichen thermischen Desinfektion oder Sterilisation in Strömungsrichtung des Sterilisationsmediums oder des Desinfektionsmediums vor oder hinter dem zu sterilisierenden Hohlkörperobjekt angeordnet ist. Das heißt, es ist möglich, dass im bestimmungsgemäßen Betrieb der Vorrichtung ein Sterilisationsmedium oder Desinfektionsmedium zuerst die Vorrichtung und dann das zu desinfizierende oder zu sterilisierende Hohlkörperobjekt oder zuerst das zu desinfizierende oder zu sterilisierende Hohlkörperobjekt und dann die Vorrichtung durchströmt.

Durch eine derartige unmittelbare Verbindung zwischen der Vorrichtung und dem zu desinfizierenden oder zu sterilisierenden Hohlkörperobjekt wird nur ein minimaler Bauraum für die Vorrichtung benötigt. Darüber hinaus wird kein Aufbereitungsplatz für ein zu desinfizierendes oder zu sterilisierendes Hohlkörperobjekt für die Vorrichtung verbraucht. Vielmehr können durch eine derartige Ausgestaltung der Vorrichtung in einem Gerät zur thermischen Desinfektion oder Sterilisation genauso viele Hohlkörperobjekte desinfiziert oder sterilisiert werden, wie wenn gar keine Vorrichtung zum Nachweis einer erfolgreichen Desinfektion oder Sterilisation eingesetzt würde. Gleichzeitig wird durch den Einsatz einer derartigen Vorrichtung jedoch der Desinfektions- oder Sterilisationserfolg ermittelt.

In einer Variante ist es vorgesehen, dass das Verbindungsmittel zum direkten Verbinden der Vorrichtung mit einem Medieneinlass eines zu desinfizierenden oder zu sterilisierenden Hohlkörperobjekts dient. Dann ist die Vorrichtung in Strömungsrichtung des Sterilisationsmediums bzw. Desinfektionsmediums vor dem zu desinfizierenden oder zu sterilisierenden Hohlkörperobjekt angeordnet. Das heißt, ein der Vorrichtung zugeführtes Prozessmedium muss dann, wenn ein Hohlkörperobjekt mit der Vorrichtung verbunden ist, zunächst durch die Prozessmedienleitung strömen, bevor es in ein Innenlumen des Hohlkörperobjekts gelangen kann. In dieser Variante kann die Vorrichtung beispielsweise als Adapter ausgestaltet sein, der auf einen Anschluss aufgebracht wird, welcher üblicherweise zur Aufnahme von zu sterilisierenden oder zu desinfizierenden Hohlkörperobjekten vorgesehen ist. Das Verbindungsmittel der Vorrichtung dient dann selbst wiederum als Adapter zur Aufnahme eines zu desinfizierenden oder zu sterilisierenden Hohlkörperobjekts. Die Vorrichtung ist dann folglich zwischen einem in einem Gerät zur thermischen Desinfektion oder Sterilisation vorgesehenen Anschluss für ein zu desinfizierendes oder zu sterilisierendes Hohlkörperobjekt und dem zu sterilisierenden oder zu desinfizierenden Hohlkörperobjekt angeordnet. Auch durch eine derartige Anordnung wird kein unnötiger Bauraum für die Vorrichtung benötigt. Darüber hinaus wird kein Aufbereitungsplatz für ein zu desinfizierendes oder zu sterilisierendes Hohlkörperobjekt für die Vorrichtung verbraucht.

In einer Variante ist die Indikatoraufnahme nur mit der Prozessmedienleitung strömungstechnisch verbunden. Dabei ist diese Verbindung durch einen einzigen Leitungsabschnitt realisiert. Die Indikatoraufnahme stellt in dieser Variante also eine strömungstechnisch mit der Prozessmedienleitung verbundene "Sackgasse" dar, aus der das Sterilisationsmedium bzw. Desinfektionsmedium nur durch denselben Leitungsabschnitt ausströmen kann, über den es in die Indikatoraufnahme eingeströmt ist. Mittels einer solchen Anordnung lässt sich ein optionales strömungstechnisches Absperren der Indikatoraufnahme gegenüber der Prozessmedienleitung besonders einfach realisieren.

In einer Variante weist die Vorrichtung ein Ventil auf, durch das eine Strömungsverbindung zwischen der Prozessmedienleitung und der Indikatoraufnahme wahlweise hergestellt werden kann oder unterbrochen werden kann.

Durch ein derartiges Ventil ist es möglich, dass ein Kontakt zwischen einem durch die Prozessmedienleitung strömenden Prozessmedium, das nicht der eigentlichen Desinfektion oder Sterilisation dient, und einem in der Indikatoraufnahme aufgenommenen Indikator effektiv unterbunden wird. Denn wenn ein solches Prozessmedium durch die Prozessmedienleitung strömt, bleibt das Ventil geschlossen. Nur dann, wenn durch die Prozessmedienleitung ein der Sterilisation oder Desinfektion dienendes Prozessmedium, also ein Sterilisationsmedium oder ein Desinfektionsmedium, strömt, öffnet sich das Ventil, sodass das Sterilisationsmedium oder Desinfektionsmedium mit einem Indikator, der in der Indikatoraufnahme der Vorrichtung aufgenommen ist, in Kontakt kommt.

Durch eine derartige Ausgestaltung der Vorrichtung zum Nachweis des Erfolges einer thermischen Sterilisation oder Desinfektion werden falsch-positive Resultate signifikant reduziert, da es aufgrund des Ventils nicht zu einer unbeabsichtigten Kontaktierung eines in der Indikatoraufnahme aufgenommenen Indikators durch ein nicht der eigentlichen Sterilisation oder Desinfektion dienendes Prozessmedium kommen kann.

Das Ventil kann in einer Variante auch als aktiv schaltendes Ventil bezeichnet werden.

In einer Variante wird das Ventil durch ein externes Steuersignal gesteuert. Ein solches Steuersignal kann beispielsweise von einer Steuerungseinheit eines Geräts zur thermischen Desinfektion oder Sterilisation, in das die Vorrichtung zum Nachweis einer erfolgreichen thermischen Desinfektion oder Sterilisation eingebaut ist, ausgelöst werden. Das heißt, über einen definierten Ablauf eines Aufbereitungsprogramms von zu desinfizierenden oder zu sterilisierenden Objekten lässt sich zu einem bestimmten Programmzeitpunkt ein Öffnen bzw. Schließen des Ventils erreichen, um eine Verbindung zwischen der Indikatoraufnahme und der Prozessmedienleitung herzustellen oder zu unterbrechen. Mithin dient das externe Steuersignal dazu, das Ventil von seinem geschlossenen Zustand in seinen geöffneten Zustand oder aber von seinen geöffneten Zustand in seinen geschlossenen Zustand zu überführen. Das Steuersignal kann auch als Schaltsignal bezeichnet werden.

Um eine von einem konkreten Aufbereitungsprogramm unabhängige, aber dennoch zuverlässige Schaltung des Ventils zu erreichen, ist es in einer Variante vorgesehen, dass das Ventil ein thermisch schaltendes Ventil ist. Bei einer Temperatur, die oberhalb einer Schalttemperatur eines solchen thermisch schaltenden Ventils liegt, schaltet das Ventil selbstständig (ohne weiteres Steuersignal) in seinen geöffneten Zustand. Dabei ist in einer Variante auch vorgesehen, dass das Ventil bei einer Temperatur, die unterhalb der Schalttemperatur des thermisch schaltenden Ventils liegt, selbstständig wieder in seinen geschlossenen Zustand schaltet.

Ein solches thermisch schaltendes Ventil lässt sich also beliebig oft öffnen (in seinen geöffneten Zustand überführen) und schließen (in seinen geschlossenen Zustand überführen), und zwar in Abhängigkeit der Temperatur des Ventils. Diese Temperatur wird durch die Temperatur eines durch die Prozessmedienleitung strömenden Prozessmediums beeinflusst bzw. vorgegeben.

In einer Variante liegt die Schalttemperatur in einem Temperaturbereich von 70 °C bis 150 °C, insbesondere von 75 °C bis 145 °C, insbesondere von 80 °C bis 140 °C, insbesondere von und 85 °C bis 135 °C, insbesondere von 90 °C bis 130 °C, insbesondere von 95 °C bis 125 °C, insbesondere von 96 °C bis 120 °C, insbesondere von 97 °C bis 115 °C, insbesondere von 98 °C bis 110 °C, insbesondere von 99 °C bis 105 °C, insbesondere von 90 °C bis 100 °C.

Durch eine Schalttemperatur in diesem Temperaturbereich bleibt das Ventil im bestimmungsgemäßen Betrieb der Vorrichtung geschlossen, wenn zur Aufbereitung von Hohlkörperinstrumenten eingesetzte Prozessmedien durch die Prozessmedienleitung strömen. Denn diese Prozessmedien haben typischerweise eine Temperatur zwischen 10 °C (beispielsweise kaltes Wasser zum Vorspülen) bis zu unter 70 °C (heiße Reinigungsflüssigkeit zur intensiven Reinigung der aufzubereitenden Objekte). Demgegenüber weist ein zur thermischen Sterilisation oder Desinfektion eingesetztes Prozessmedium eine Temperatur von 70 °C oder mehr auf. Häufig wird zu Sterilisation oder Desinfektion Wasserdampf eingesetzt, der (unter Normaldruck) eine Temperatur von 100 °C aufweist. Wenn ein solches Sterilisationsmedium oder Desinfektionsmedium durch die Prozessmedienleitung strömt, öffnet das Ventil in einer Variante selbstständig, sodass das Sterilisationsmedium oder Desinfektionsmedium in die Indikatoraufnahme gelangen kann und mit einem dort angeordneten Indikator in Wechselwirkung treten kann.

Um ein selbsttätiges Schalten des als thermisch schaltenden Ventils ausgestalteten Ventils zu erreichen, weist das Ventil in einer Variante einen Aktor bzw. ein Stellelement auf, das ein Formgedächtnismaterial oder ein Bimetall aufweist. In einer Variante besteht das Stellelement aus dem Formgedächtnismaterial oder dem Bimetall. Besonders geeignete Formgedächtnismaterialien sind Formgedächtnismaterialien mit Zweiweg-Effekt, also Formgedächtnismaterialien, die sich dauerhaft an zwei unterschiedliche Formen "erinnern" können, nämlich eine Form bei hoher Temperatur und eine Form bei niedriger Temperatur. Formgedächtnislegierungen und Formgedächtniskunststoffe sind geeignete Formgedächtnismaterialien. Das Stellelement kann beispielsweise als Feder realisiert sein.

Insbesondere dann, wenn das Stellelement ein Formgedächtnismaterial aufweist, weist das Ventil in einer Variante eine Gegenfeder auf. Diese dient dazu, das Formgedächtnismaterial wieder in seine Ausgangsposition zurückzudrücken. Denn im erkalteten Zustand bewegt sich das Formgedächtnismaterial nicht von alleine in seine Ausgangsposition zurück. Es wird nur "weich" und kann mit einer geringeren Kraft in seine Ausgangslage zurückbewegt werden.

Das Ventil kann in einer Variante einen thermischen Aktor, eine Gegenfeder aus Federstahl oder einem vergleichbaren Material und einen Stößel aufweisen. Der thermische Aktor kann beispielsweise in Form einer Feder aus einer Formgedächtnislegierung oder einem anderen Formgedächtnismaterial realisiert sein. Bei einer Temperatur unterhalb der Schalttemperatur des Ventils ist das Ventil geschlossen. Der Stößel drückt dann auf eine Dichtkontur des Ventils. Sämtliche Prozessmedien strömen dann in der Prozessmedienleitung durch die Vorrichtung, ohne in die Indikatoraufnahme zu gelangen. Wird die Schalttemperatur überschritten, beginnt sich der thermische Aktor zu verformen. Im konkreten Beispielsfall dehnt sich der Aktor aus und öffnet das Ventil. Das Prozessmedium, das mit einer Temperatur oberhalb der Schalttemperatur durch die Prozessmedienleitung geführt wird und somit auch für eine Erwärmung des Ventils auf eine Temperatur oberhalb der Schalttemperatur verantwortlich ist, kann dann das Ventil passieren, in die Indikatoraufnahme strömen und mit dem dort angeordneten Indikator interagieren. Bei diesem Prozessmedium handelt es sich insbesondere um ein Desinfektionsmedium oder ein Sterilisationsmedium wie beispielsweise Wasserdampf.

In einer Variante weist die Vorrichtung zusätzlich eine Antriebsmedienleitung auf. Diese Antriebsmedienleitung ist dabei strömungstechnisch sowohl von der Prozessmedienleitung als auch von der Indikatoraufnahme getrennt. Durch die Antriebsmedienleitung kann beispielsweise Öl geführt werden, das in Antriebskanäle eines aufzubereitenden Hohlkörperobjekts eingebracht werden kann. Durch das Vorsehen einer derartigen Antriebsmedienleitung lässt sich die Vorrichtung besonders einfach in bestehende Aufbereitungssysteme integrieren.

Ein Aspekt der vorliegenden Erfindung betrifft eine Anordnung aus einer Vorrichtung zum Nachweis einer erfolgreichen thermischen Desinfektion oder Sterilisation gemäß den obigen Erläuterungen und einem mit dieser Vorrichtung verbundenen zu desinfizierenden oder zu sterilisierenden Hohlkörperobjekt. Dabei weist die Vorrichtung eine Prozessmedienleitung und eine mit der Prozessmedienleitung strömungstechnisch verbundene Indikatoraufnahme auf. Die Indikatoraufnahme dient zur Aufnahme eines Indikators, der wiederum zum Anzeigen des Erfolgs einer thermischen Desinfektion oder Sterilisation dient.

Diese Anordnung zeichnet sich erfindungsgemäß dadurch aus, dass das Hohlkörperobjekt mittels eines Verbindungsmittels direkt (unmittelbar) mit der Vorrichtung verbunden ist. Durch diese Verbindung wird eine Strömungsverbindung zwischen der Prozessmedienleitung der Vorrichtung und einem Innenlumen des Hohlkörperobjekts hergestellt. Dadurch wird ein Prozessmedium, das der Vorrichtung zugeführt wird, sowohl durch die Prozessmedienleitung der Vorrichtung als auch durch das Innenlumen des Hohlkörperobjekts geleitet.

In einer Variante ist es vorgesehen, dass das Verbindungsmittel direkt mit einem Medieneinlass (also einem proximalen Anschluss eines Hohlkörperobjekts, der dazu vorgesehen ist, dass ein Medium in ihn eingebracht werden kann, insbesondere damit es an anderer Stelle aus dem Hohlkörperobjekt wieder austreten kann) des zu desinfizierenden oder zu sterilisierenden Hohlkörperobjekts verbunden ist. Dann ist die Vorrichtung in der Anordnung in Strömungsrichtung des Sterilisationsmediums bzw. Desinfektionsmediums vor dem zu desinfizierenden oder zu sterilisierenden Hohlkörperobjekt angeordnet. Das heißt, ein der Vorrichtung zugeführtes Prozessmedium muss dann, wenn ein Hohlkörperobjekt mit der Vorrichtung verbunden ist, zunächst durch die Prozessmedienleitung strömen, bevor es in das Innenlumen des Hohlkörperobjekts gelangen kann. In dieser Variante kann die Vorrichtung beispielsweise als Adapter ausgestaltet sein, der auf einen Anschluss aufgebracht (beispielsweise aufgeschraubt oder aufgesteckt) wird, welcher üblicherweise zur Aufnahme von zu sterilisierenden oder zu desinfizierenden Hohlkörperobjekten vorgesehen ist. Das Verbindungsmittel der Vorrichtung dient dann selbst wiederum als Adapter zur Aufnahme des zu desinfizierenden oder zu sterilisierenden Hohlkörperobjekts. Die Vorrichtung ist dann folglich zwischen einem in einem Gerät zur thermischen Desinfektion oder Sterilisation vorgesehenen Anschluss für ein zu desinfizierendes oder zu sterilisierendes Hohlkörperobjekt und dem zu sterilisierenden oder zu desinfizierenden Hohlkörperobjekt angeordnet.

In einer Variante weist die Vorrichtung einen Strömungswiderstand auf, der größer oder gleich dem Strömungswiderstand des Innenlumens des mit der Vorrichtung verbundenen Hohlkörperobjekts ist. Da die Innengeometrien der mit der Vorrichtung zu verbindenden Hohlkörperobjekte regelmäßig gut bekannt sind, lassen sich in dieser Variante beispielsweise unterschiedliche Vorrichtungen bereithalten, die an die Strömungswiderstände unterschiedlicher Hohlkörperobjekt angepasst sind. Wenn beispielsweise ein Hohlkörperobjekt mit einem Innenlumen kleinen Durchmessers desinfiziert oder sterilisiert werden soll, wird in dieser Variante eine Vorrichtung eingesetzt, die einen großen Strömungswiderstand aufweist. Soll demgegenüber ein Hohlkörperobjekt mit einem Innenlumen großen Durchmessers desinfiziert oder sterilisiert werden, wird in dieser Variante eine Vorrichtung eingesetzt, die einen kleinen Strömungswiderstand aufweist. Auf diese Weise ist sichergestellt, dass ein Desinfektionsmedium oder Sterilisationsmedium nicht leichter in die Indikatoraufnahme als durch das Innenlumen des mit der Vorrichtung verbundenen Hohlkörperobjekts strömen kann. Dadurch werden falsch-positive Ergebnisse der Vorrichtung im Hinblick auf den Erfolg der durchgeführten Desinfektion oder Sterilisation vermieden.

In einer Variante ist das Hohlkörperobjekt ausgewählt aus der Gruppe bestehend aus medizinischen oder zahnmedizinischen Instrumenten, medizinischen oder zahnmedizinischen Übertragungsinstrumenten und Endoskopen. Beispiele für medizinische oder zahnmedizinische Instrumente sind Bohrer, Haken und Absaugstutzen. Beispiele für medizinische oder zahnmedizinische Übertragungselemente sind Turbinen, Winkelstücke und Handstücke.

Ein Aspekt der vorliegend beanspruchten Erfindung betrifft ein Gerät zur thermischen Desinfektion oder Sterilisation von Hohlkörperobjekten mit den nachfolgend erläuterten Merkmalen. Ein solches Gerät weist eine Prozessmedienzuführung auf, durch die zumindest ein für eine thermische Desinfektion oder Sterilisation benötigtes Prozessmedium geleitet werden kann. Typischerweise werden durch eine derartige Prozessmedienzuführung zahlreiche unterschiedliche Prozessmedien geleitet.

Das Gerät zeichnet sich erfindungsgemäß dadurch aus, dass es eine Vorrichtung zum Nachweis einer erfolgreichen thermischen Desinfektion oder Sterilisation mit den vorstehend erläuterten Merkmalen umfasst. Dabei steht eine Prozessmedienleitung der Vorrichtung zum Nachweis einer erfolgreichen thermischen Desinfektion oder Sterilisation in Strömungsverbindung mit der Prozessmedienzuführung des Geräts zur thermischen Desinfektion oder Sterilisation. Mithin strömen Prozessmedien, die durch die Prozessmedienzuführung geleitet werden, dann im Betrieb des Gerätes auch durch die Prozessmedienleitung der Vorrichtung zum Nachweis einer erfolgreichen thermischen Desinfektion oder Sterilisation.

Dabei ist die Vorrichtung vorzugsweise entfernbar in dem Gerät angeordnet, sodass sie beispielsweise bei Bedarf gegen eine baugleiche Vorrichtung ausgetauscht werden kann. Dabei ist es insbesondere vorgesehen, dass die Vorrichtung auf einen Anschluss, der zur Aufnahme eines zu desinfizierenden oder zu sterilisierenden Hohlkörperobjekts in dem Gerät ausgebildet ist, aufgesteckt oder aufgeschraubt wird.

Da die Vorrichtung selbst zur Herstellung einer Verbindung mit einem zu desinfizierenden oder zu sterilisierenden Hohlkörperobjekt vorgesehen und eingerichtet ist, also ein entsprechendes Verbindungsmittel umfasst, kann ein zu desinfizierendes oder zu sterilisierendes Hohlkörperobjekt beispielsweise unter Zwischenschaltung der Vorrichtung zum Nachweis einer erfolgreichen thermischen Desinfektion oder Sterilisation an seinem üblichen Platz innerhalb des Geräts zur thermischen Desinfektion oder Sterilisation aufgenommen werden.

In einer Variante ist das Gerät als Autoklav, als Kombinationsautoklav oder als Reinigungs- und Desinfektionsgerät ausgestaltet. In Kombinationsautoklaven und in Reinigungs- und Desinfektionsgeräten durchlaufen zu desinfizierende oder zu sterilisierende Hohlkörperobjekte während der Aufbereitung mehrere Prozessschritte, in denen sie mit unterschiedlichen Prozessmedien in Berührung kommen. So lässt sich ein in einem Kombinationsautoklaven durchgeführtes Aufbereitungsverfahren von Hohlkörperobjekten beispielsweise in die vier nachfolgend erläuterten, nacheinander ablaufenden Prozessschritte unterteilen.

Zunächst erfolgen eine Reinigung, dann eine Pflege, anschließend eine Sterilisation und dann eine Trocknung der zu sterilisierenden Hohlkörperobjekte. Während der Reinigung werden die Hohlkörperobjekte mit voll entsalztem Wasser (VE-Wasser) gereinigt. Das Wasser wird dabei durch verschiedene Strömungsführungen sowohl zur Außenreinigung als auch zur Innenreinigung der Hohlkörperobjekte eingesetzt. Dabei wird im Rahmen der Reinigung zunächst kaltes VE-Wasser (mit einer Temperatur von weniger als 50 °C) und später warmes VE-Wasser (mit einer Temperatur von 50 °C oder mehr), optional unter Zusatz eines Reinigungsmittels, verwendet.

Nach der Reinigung findet ein Pflegevorgang statt. Dabei werden die Innengeometrien der Hohlkörperobjekte mit Pflegeöl versorgt. Wenn zahnärztliche Hohlkörperinstrumente als Hohlkörperobjekte aufbereitet werden, bietet sich für diesen Schritt der Einsatz dentalen Pflegeöls an.

Anschließend startet die eigentliche Sterilisation. Dabei wird ein Sterilisationsmedium wie beispielsweise Wasserdampf sowohl in die Sterilisationskammer (und damit auf die Außenseiten der zu sterilisierenden Hohlkörperobjekte) als auch durch die Innengeometrien der Hohlkörperobjekte geleitet. Nach Abschluss der Sterilisation erfolgt noch ein Trocknungsvorgang, in dem Restfeuchtigkeit von den Hohlkörperobjekten entfernt wird. Somit strömen während der Reinigung, der Pflege, der Sterilisation und der Trocknung unterschiedliche Prozessmedien durch die Innengeometrien der Hohlkörperobjekte und der Prozessmedienleitung der Vorrichtung zum Nachweis einer erfolgreichen thermischen Desinfektion oder Sterilisation. Das aktive schaltende Ventil dieser Vorrichtung stellt dabei sicher, dass von diesen Prozessmedien lediglich das Sterilisationsmedium, beispielsweise Wasserdampf, in die Indikatoraufnahme der Vorrichtung gelangt und mit einem dort vorhandenen Indikator wechselwirken kann.

Ein Aspekt der vorliegend beanspruchten Erfindung betrifft ein Verfahren zur thermischen Desinfektion oder Sterilisation von Hohlkörperobjekten mit den nachfolgend erläuterten Merkmalen. Dieses Verfahren zeichnet sich durch die Verwendung einer Vorrichtung zum Nachweis einer erfolgreichen thermischen Desinfektion oder Sterilisation gemäß den vorherigen Erläuterungen aus. Eine solche Vorrichtung weist eine Prozessmedienleitung und eine mit der Prozessmedienleitung strömungstechnisch verbundene Indikatoraufnahme auf. In der Indikatoraufnahme ist ein Indikator angeordnet, der zum Anzeigen des Erfolgs der thermischen Desinfektion oder Sterilisation dient. Der Indikator kann beispielsweise ein Chemoindikator oder ein Bioindikator sein. Darüber hinaus weist die Vorrichtung ein Verbindungsmittel zum direkten Verbinden mit einem zu desinfizierenden oder zu sterilisierenden Hohlkörperobjekt auf. Dabei weist das Verfahren die nachfolgend erläuterten Schritte auf.

Zunächst wird das Hohlkörperobjekt mit der Vorrichtung mittels des Verbindungsmittels verbunden. Dadurch wird eine Strömungsverbindung zwischen der Prozessmedienleitung der Vorrichtung und einem Innenlumen des Hohlkörperobjekts ausgebildet.

Anschließend wird ein Desinfektionsmedium oder Sterilisationsmedium, also ein für eine thermische Desinfektion oder Sterilisation benötigtes Prozessmedium, sowohl durch die Prozessmedienleitung der Vorrichtung als auch durch das Innenlumen des Hohlkörperobjekts geleitet.

Das vorstehend erläuterte Verfahren kann Teil eines Aufbereitungsverfahrens sein, das beispielsweise in einem Reinigungs- und Desinfektionsgerät oder in einem Kombinationsautoklaven durchgeführt wird. Es kann auch ein Verfahren sein, das in einem Autoklaven durchgeführt wird.

In einer Variante umfasst das Desinfektionsmedium oder Sterilisationsmedium Wasserdampf. Beispielsweise kann es sich beim Desinfektionsmedium oder Sterilisationsmedium (ausschließlich) um Wasserdampf handeln.

In einer Variante wird das Verfahren derart durchgeführt, dass das Prozessmedium zum Erreichen des Innenlumens des Hohlkörperobjekts durch die Prozessmedienleitung strömen muss. Das heißt, die Vorrichtung zum Nachweis einer erfolgreichen Sterilisation oder Desinfektion ist in dieser Variante in Strömungsrichtung vor dem zu desinfizierenden oder zu sterilisierenden Hohlkörperobjekt angeordnet. Dabei ist es insbesondere vorgesehen, dass die Vorrichtung als Adapter zum Verbinden eines zu desinfizierenden oder zu sterilisierenden Hohlkörperobjekts und einem Aufnahmeplatz für dieses Hohlkörperobjekt in einem Gerät zur thermischen Desinfektion oder Sterilisation ausgestaltet ist.

Im Rahmen dieses Verfahrens kann beispielsweise eine Vorrichtung eingesetzt werden, bei der zwischen der Indikatoraufnahme und der Prozessmedienleitung ein Ventil vorgesehen ist, mit dem eine Strömungsverbindung zwischen der Indikatoraufnahme der Prozessmedienleitung hergestellt werden oder unterbrochen werden kann. In dieser Variante weist das Verfahren zusätzlich die nachfolgend erläuterten Schritte auf.

Zunächst wird das Ventil in seinem geschlossenen Zustand gehalten, solange kein Desinfektionsmedium oder Sterilisationsmedium (also kein für eine thermische Desinfektion oder Sterilisation benötigtes Prozessmedium) durch die Prozessmedienleitung strömt.

Wenn das Desinfektionsmedium oder Sterilisationsmedium in das Innenlumen des zu desinfizierenden oder zu sterilisierenden Hohlkörperobjekts und gleichzeitig in die Prozessmedienleitung geleitet wird, wird das Ventil automatisch von seinem geschlossenen Zustand in seinen geöffneten Zustand überführt.

Anschließend wird das Ventil automatisch von seinem geöffneten Zustand in seinen geschlossenen Zustand überführt, wenn kein Desinfektionsmedium oder Sterilisationsmedium mehr durch die Prozessmedienleitung strömt.

Durch diese Vorgehensweise wird in dieser Variante sichergestellt, dass nur das Desinfektionsmedium oder Sterilisationsmedium mit dem in der Indikatoraufnahme angeordneten Indikator in Kontakt tritt. Wenn der Indikator später ausgelesen bzw. ausgewertet wird, lässt sich das Auslese- oder Auswerteergebnis also unmittelbar auf die Einwirkung des Desinfektionsmediums oder des Sterilisationsmediums zurückführen, während ein möglicher Einfluss anderer Prozessmedien auf den Indikator ausgeschlossen werden kann, da diese anderen Prozessmedien gar nicht mit dem Indikator in Kontakt gekommen sind.

Sämtliche der vorstehend erläuterten Varianten und bevorzugten Ausgestaltungen sind in beliebiger Weise miteinander kombinierbar. Ferner sind Varianten und Ausgestaltungen der beschriebenen Vorrichtung zum Nachweis einer erfolgreichen thermischen Desinfektion oder Sterilisation einzeln oder in beliebiger Kombination auf die beschriebene Anordnung, auf das beschriebene Gerät und/oder das beschriebene Verfahren übertragbar. Ferner sind Varianten und Ausgestaltungen der beschriebenen Anordnung einzeln oder in beliebiger Kombination auf die beschriebene Vorrichtung, das beschriebene Gerät und/oder das beschriebene Verfahren übertragbar. Außerdem sind Varianten und Ausgestaltungen des beschriebenen Geräts einzeln oder in beliebiger Kombination auf die beschriebene Vorrichtung, die beschriebene Anordnung und/oder das beschriebene Verfahren übertragbar. Schließlich sind Varianten und Ausgestaltungen des beschriebenen Verfahrens einzeln oder in beliebiger Kombination auf die beschriebene Vorrichtung, die beschriebene Anordnung und/oder das beschriebene Gerät übertragbar.

Weitere Einzelheiten von Aspekten der vorliegend beschriebenen Erfindung werden anhand eines Ausführungsbeispiels und entsprechender Figuren näher erläutert. Es zeigen:
- Figur 1: ein Ausführungsbeispiel einer Vorrichtung zum Nachweis einer erfolgreichen thermischen Desinfektion oder Sterilisation mit einem daran angeschlossenen Hohlkörperinstrument und
- Figur 2: eine schematische Darstellung eines Ausführungsbeispiels einer Vorrichtung zum Nachweis einer erfolgreichen thermischen Desinfektion oder Sterilisation mit einem damit verbundenen Hohlkörperinstrument.

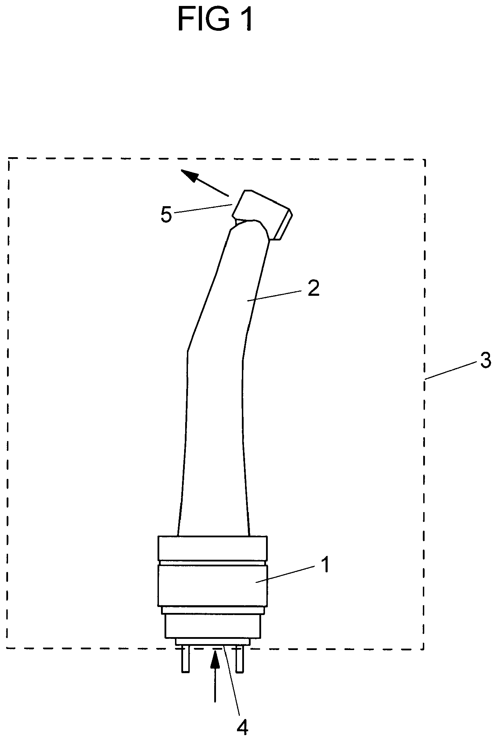

Die Figur 1 zeigt einen Prüfkörper 1, der als Vorrichtung zum Nachweis einer erfolgreichen thermischen Desinfektion oder Sterilisation dient. Mit dem Prüfkörper 1 direkt verbunden ist ein Hohlkörperinstrument 2, das als Hohlkörperobjekt dient. Im Inneren des Hohlkörperinstruments 2 ist ein Lumen ausgebildet, durch das ein Sterilisationsmedium strömen soll, um eine Sterilisation dieses Innenlumens zu erreichen.

Der Prüfkörper 1 und das Hohlkörperinstrument 2 sind in einer Sterilisationskammer 3 eines Sterilisationsgerätes angeordnet.

Im Innern des Prüfkörpers 1 ist eine Prozessmedienleitung ausgebildet, sodass ein Prozessmedium, das durch einen Prozessmedieneinlass 4 in den Prüfkörper 1 eintritt, diesen Prüfkörper 1 durchströmt und anschließend in das Innenlumen des Hohlkörperinstruments 2 eintritt. Anschließend durchströmt es das gesamte Innenlumen des Hohlkörperinstruments 2 und tritt am Prozessmedienauslass 5 wieder aus dem Hohlkörperinstrument 2 aus. Der Weg eines Prozessmediums durch den Prüfkörper 1 und das Hohlkörperinstrument 2 ist in der Figur 1 durch entsprechende Pfeile visualisiert.

Wie aus der in der Figur 1 dargestellten spezifischen Anordnung von Prüfkörper 1 und Hohlkörperinstrument 2 hervorgeht, ist der Prüfkörper 1 als Adapter ausgebildet, an den das Hohlkörperinstrument 2 angeschlossen werden kann. Der Prüfkörper 1 wird selbst an einem Aufnahmeplatz innerhalb eines Sterilisationsgerätes angeordnet, der typischerweise zur unmittelbaren Aufnahme des Hohlkörperinstruments 2 dienen würde. Zu diesem Zweck wird der Prüfkörper 1 auf den entsprechenden Aufnahmeplatz aufgesteckt oder aufgeschraubt oder anderweitig mit dem Aufnahmeplatz verbunden. Da der Prüfkörper 1 durch seine Ausbildung als Adapter weiterhin den Anschluss des Hohlkörperinstruments 2 ermöglicht, geht durch den Einsatz des Prüfkörpers 1 keine Sterilisationskapazität innerhalb Sterilisationsgerätes verloren. Vielmehr wird der Prüfkörper 1 einfach zwischen einen geräteseitigen Prozessmedienauslass und einen Prozessmedieneinlass in das Hohlkörperinstrument 2 geschaltet.

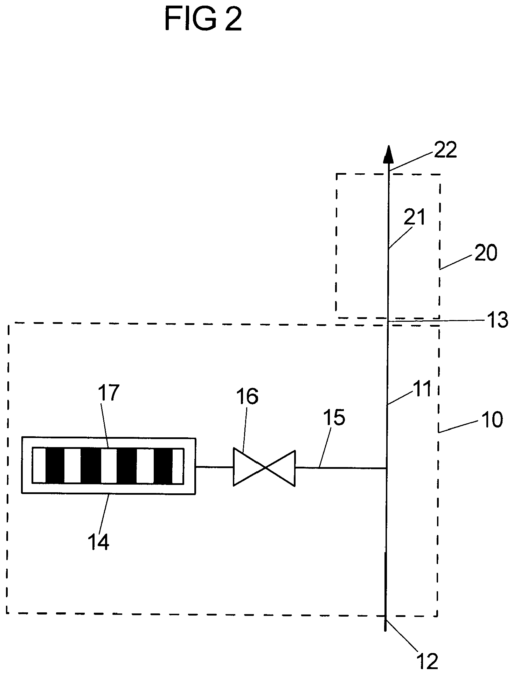

Die Figur 2 zeigt eine schaltbildartige Darstellung eines Prüfkörpers 10 und eines mit dem Prüfkörper 10 direkt verbundenen Hohlkörperinstrument 20. Dabei dient der Prüfkörper 10 als Vorrichtung zum Nachweis einer erfolgreichen thermischen Desinfektion oder Sterilisation, wäre das Hohlkörperinstrument 20 als zu desinfizierendes oder zu sterilisierendes Hohlkörperobjekt dient.

Im Prüfkörper 10 ist eine Prozessmedienleitung 11 ausgebildet, durch die ein Prozessmedium vom Prozessmedieneinlass 12 zu einem Übergang 13 zum Hohlkörperinstrument 20 strömen kann. Wenn das Hohlkörperinstrument 20 - wie in der Figur 2 dargestellt - mit dem Prüfkörper 10 verbunden ist, steht die Prozessmedienleitung 11 in Strömungsverbindung mit einem Innenlumen 21 des Hohlkörperinstruments 20. Dieses Innenlumen 21 erstreckt sich bis zu einem Prozessmedienauslass 22, sodass ein Prozessmedium vom Prozessmedieneinlass 12 des Prüfkörpers 10 bis zum Prozessmedienauslass 22 des Hohlkörperinstruments 20 geführt wird.

Innerhalb des Prüfkörpers 10 ist eine Indikatoraufnahme 14 ausgebildet, die über eine Indikatoraufnahmeleitung 15 strömungstechnisch mit der Prozessmedienleitung 11 verbunden ist. Dabei stellt die Indikatoraufnahmeleitung 15 die einzige strömungstechnische Verbindung zwischen der Indikatoraufnahme 14 und der Prozessmedienleitung 11 dar. Innerhalb der Indikatoraufnahmeleitung 15 ist ein Ventil 16 angeordnet, das als aktiv schaltendes Ventil ausgestaltet ist. Mittels des Ventils 16 lässt sich eine Strömungsverbindung zwischen der Prozessmedienleitung 11 und der Indikatoraufnahme 14 herstellen (das Ventil 16 ist dann in seinem geöffneten Zustand) oder unterbrechen (das Ventil 16 ist dann in seinem geschlossenen Zustand).

In der Indikatoraufnahme 14 ist zudem ein Indikator 17 angeordnet, der als Indikator zum Anzeigen des Erfolgs in einer thermischen Desinfektion oder Sterilisation dient. Wenn der Indikator 17 mit Wasserdampf, der als Sterilisationsmedium eingesetzt wird, in Berührung kommt, verfärbt er sich nach einer vordefinierten Inkubationszeit, sodass mittels des entsprechenden Farbumschlag des Indikators 17 festgestellt werden kann, ob der Wasserdampf die erforderliche Mindestzeit durch die Prozessmedienleitung 11 und damit durch das Innenlumen 21 geströmt ist.

Zu diesem Zweck ist das Ventil 16 immer dann geöffnet, wenn Wasserdampf durch die Prozessmedienleitung 11 strömt. Strömt hingegen ein anderes Prozessmedium durch die Prozessmedienleitung 11, ist das Ventil 16 geschlossen.

## Patentansprüche

1. Vorrichtung zum Nachweis einer erfolgreichen thermischen Desinfektion oder Sterilisation, mit einer Prozessmedienleitung (11) und einer mit der Prozessmedienleitung (11) strömungstechnisch verbundenen Indikatoraufnahme (14) zur Aufnahme eines Indikators (17), der zum Anzeigen des Erfolgs einer thermischen Desinfektion oder Sterilisation dient,
**dadurch gekennzeichnet,**
**dass** die Vorrichtung (1, 10) ein Verbindungsmittel zum direkten Verbinden mit einem zu desinfizierenden oder zu sterilisierenden Hohlkörperobjekt (2, 20) unter Ausbildung einer Strömungsverbindung zwischen der Prozessmedienleitung (11) und einem Innenlumen (21) des Hohlkörperobjekts (2, 20) aufweist, so dass ein der Vorrichtung (1, 10) zugeführtes Prozessmedium dann, wenn ein Hohlkörperobjekt (2, 20) mit der Vorrichtung (1, 10) verbunden ist, sowohl durch die Prozessmedienleitung (11) der Vorrichtung (1, 10) als auch durch das Innenlumen (21) des Hohlkörperobjekts (2, 20) geleitet wird.

2. Vorrichtung nach Anspruch 1, **dadurch gekennzeichnet, dass** das Verbindungsmittel zum Verbinden mit einem Medieneinlass eines zu desinfizierenden oder zu sterilisierenden Hohlkörperobjekts (2, 20) dient, so dass ein der Vorrichtung (1, 10) zugeführtes Prozessmedium dann, wenn ein Hohlkörperobjekt (2, 20) mit der Vorrichtung (1, 10) verbunden ist, zunächst durch die Prozessmedienleitung (11) der Vorrichtung (1, 10) strömen muss, bevor es das Innenlumen (21) des Hohlkörperobjekts (2, 20) erreichen kann.

3. Vorrichtung nach Anspruch 1 oder 2, **dadurch gekennzeichnet, dass** die Indikatoraufnahme (14) ausschließlich mit der Prozessmedienleitung (11) strömungstechnisch verbunden ist, wobei eine Verbindung zwischen der Indikatoraufnahme (14) und der Prozessmedienleitung (11) mittels eines einzigen Leitungsabschnitts (15) realisiert ist.

4. Vorrichtung nach einem der vorherigen Ansprüche, **dadurch gekennzeichnet, dass** die Vorrichtung ein Ventil (16) aufweist, mittels dessen sich eine Strömungsverbindung zwischen der Prozessmedienleitung (11) und der Indikatoraufnahme (14) herstellen oder unterbrechen lässt.

5. Vorrichtung nach Anspruch 4, **dadurch gekennzeichnet, dass** das Ventil (16) durch ein externes Steuersignal von seinem geschlossenen Zustand in seinen geöffneten Zustand und von seinem geöffneten Zustand in seinen geschlossenen Zustand überführt werden kann.

6. Vorrichtung nach Anspruch 4, **dadurch gekennzeichnet, dass** das Ventil (16) ein thermisch schaltendes Ventil ist, das bei einer Temperatur oberhalb einer Schalttemperatur selbsttätig in seinen geöffneten Zustand schaltet.

7. Anordnung aus einer Vorrichtung zum Nachweis einer erfolgreichen thermischen Desinfektion oder Sterilisation (1, 10) nach einem der vorherigen Ansprüche und einem mit dieser Vorrichtung (1, 10) verbundenen zu desinfizierenden oder zu sterilisierenden Hohlkörperobjekt (2, 20), wobei die Vorrichtung (1, 10) eine Prozessmedienleitung (11) und eine mit der Prozessmedienleitung (11) strömungstechnisch verbundene Indikatoraufnahme (14) zur Aufnahme eines Indikators (17), der zum Anzeigen des Erfolgs einer thermischen Desinfektion oder Sterilisation dient, aufweist,
**dadurch gekennzeichnet,**
das Hohlkörperobjekt (2, 20) mittels eines Verbindungsmittels unter Ausbildung einer Strömungsverbindung zwischen der Prozessmedienleitung (11) und einem Innenlumen (21) des Hohlkörperobjekts (2, 20) direkt mit der Vorrichtung verbunden ist, so dass ein der Vorrichtung (1, 10) zugeführtes Prozessmedium sowohl durch die Prozessmedienleitung (11) der Vorrichtung (1, 10) als auch durch das Innenlumen (21) des Hohlkörperobjekts (2, 20) geleitet wird.

8. Anordnung nach Anspruch 7, **dadurch gekennzeichnet, dass** ein Medieneinlass des Hohlkörperobjekts (2, 20) mittels des Verbindungsmittels mit der Vorrichtung (1, 10) verbunden ist, so dass ein der Vorrichtung (1, 10) zugeführtes Prozessmedium zunächst durch die Prozessmedienleitung (11) der Vorrichtung (1, 10) strömen muss, bevor es das Innenlumen (21) des Hohlkörperobjekts (2, 20) erreichen kann.

9. Anordnung nach Anspruch 7 oder 8, **dadurch gekennzeichnet, dass** die Vorrichtung (1, 10) einen zumindest gleich großen Strömungswiderstand wie das Innenlumen (21) des mit der Vorrichtung verbundenen Hohlkörperobjekts (2, 20) aufweist.

10. Anordnung nach einem der Ansprüche 7 bis 10, **dadurch gekennzeichnet, dass** das Hohlkörperobjekt (2, 20) ausgewählt ist aus der Gruppe bestehend aus medizinischen oder zahnmedizinischen Hohlkörperinstrumenten, medizinischen oder zahnmedizinischen Übertragungsinstrumenten und Endoskopen.

11. Gerät zur thermischen Desinfektion oder Sterilisation von Hohlkörperobjekten, mit einer Prozessmedienzuführung, durch die hindurch zumindest ein für eine thermische Desinfektion oder Sterilisation benötigtes Prozessmedium geleitet werden kann,
**dadurch gekennzeichnet,**
**dass** das Gerät eine Vorrichtung zum Nachweis einer erfolgreichen thermischen Desinfektion oder Sterilisation (1, 10) nach einem der Ansprüche 1 bis 6 aufweist, wobei eine Prozessmedienleitung (11) der Vorrichtung zum Nachweis einer erfolgreichen thermischen Desinfektion oder Sterilisation (1, 10) in Strömungsverbindung mit der Prozessmedienzuführung steht.

12. Gerät nach Anspruch 11, **dadurch gekennzeichnet, dass** es als Autoklav, als Kombinationsautoklav oder als Reinigungs- und Desinfektionsgerät ausgestaltet ist.

13. Verfahren zur thermischen Desinfektion oder Sterilisation von Hohlkörperobjekten, **gekennzeichnet durch** die Verwendung einer Vorrichtung zum Nachweis einer erfolgreichen thermischen Desinfektion oder Sterilisation (1, 10) nach einem der Ansprüche 1 bis 6, die eine Prozessmedienleitung (11) und eine mit der Prozessmedienleitung (11) strömungstechnisch verbundene Indikatoraufnahme (14) aufweist, in der ein Indikator (17), der zum Anzeigen des Erfolgs einer thermischen Desinfektion oder Sterilisation dient, vorhanden ist, wobei die Vorrichtung (1, 10) ein Verbindungsmittel zum direkten Verbinden mit einem zu desinfizierenden oder zu sterilisierenden Hohlkörperobjekt (2, 20) aufweist, wobei das Verfahren die folgenden Schritte umfasst:
a) Verbinden eines Hohlkörperobjekts (2, 20) mit der Vorrichtung (1, 10) mittels des Verbindungsmittels unter Ausbildung einer Strömungsverbindung zwischen der Prozessmedienleitung (21) und einem Innenlumen des Hohlkörperobjekts (1, 10), und
b) Leiten zumindest eines für eine thermische Desinfektion oder Sterilisation benötigten Prozessmediums sowohl durch die Prozessmedienleitung (11) der Vorrichtung (1, 10) als auch durch das Innenlumen (21) des Hohlkörperobjekts (2, 20).

14. Verfahren nach Anspruch 13, **dadurch gekennzeichnet, dass** das zur Desinfektion oder Sterilisation benötigte Prozessmedium Wasserdampf umfasst.

15. Verfahren nach Anspruch 13 oder 14, **dadurch gekennzeichnet, dass** das Prozessmedium zum Erreichen des Innenlumens (21) durch die Prozessmedienleitung (11) strömen muss.
